# EUROPÄISCHE PATENTSCHRIFT

(11) **EP 1 039 991 B1**
(45) Veröffentlichungstag und Bekanntmachung des Hinweises auf die Patenterteilung: **14.08.2002**
(21) Anmeldenummer: 98965128.6
(22) Anmeldetag: 14.12.1998
(51) Int. Cl.: B29C 33/60

(54) **VERFAHREN ZUR TRENNENDEN UND/ODER ABTRAGENDEN BEARBEITUNG VON VORGEFERTIGTEN KUNSTSTOFF-FOLIEN**
METHOD FOR MACHINING PREFORMED PLASTIC FILM BY SEPARATION AND/OR ABLATION
PROCEDE POUR USINER DES FILMS DE MATIERE PLASTIQUE PREFORMES, PAR DECOUPAGE ET/OU ABLATION

(30) Priorität: 16.12.1997 DE 19755738
(43) Veröffentlichungstag der Anmeldung: 04.10.2000
(73) Patentinhaber: ADIAM life science AG, 41812 Erkelenz (DE)
(72) Erfinder: JANSEN, Josef, D-50937 Köln (DE)
(74) Vertreter: Vomberg, Friedhelm, Dipl.-Phys.
(86) Internationale Anmeldenummer: DE9803706
(87) Internationale Veröffentlichungsnummer: WO9930884

(56) Entgegenhaltungen:
- DE-A1- 3 248 560
- US-A- 5 397 347

## Beschreibung

Die Erfindung betrifft ein Verfahren zur Herstellung von Kunststoff-Folien mit einer vorgebbaren Kontur und/oder einer gleichmäßigen oder ungleichmäßigen Dickenverteilung durch trennende und/oder abtragende Bearbeitung von vorgefertigten Kunststoff-Folien, insbesondere zur Herstellung von flexiblen Segeln für künstliche Herzklappen.

Dünne Kunststoff-Folien können durch verschiedene Verfahren, wie z.B. Spritzen, Tiefziehen, Tauchen oder auch Gießen, hergestellt werden. Soweit es sich um Folien mit einer Dicke von 500 µm und mehr handelt, bei der die Dickenverteilung über die gesamte Folienfläche keine oder nur eine untergeordnete Bedeutung hat, ist im Regelfall keine flächenhafte Nachbehandlung der Folie erforderlich, die zur Herstellung der gewünschten Kontur gestanzt wird. Anders verhält es sich hingegen bei solchen Kunststoff-Folien, bei denen eine reproduzierbar einstellbare gleichmäßige Dicke oder eine lokal definierte Dickenverteilung gewünscht wird, die im Bereich von 30 µm bis 500 µm liegen kann.

Prothetische Herzklappen bestehen aus einem Stützgehäuse mit einem Basisring, der mindestens zwei im wesentlichen in Ringachsrichtung weisende, über eine bogenförmige, der Befestigung flexibler Segel dienende Wandung verbundene Pfosten trägt, wie sie beispielsweise in der DE 38 34 545 C2 beschrieben werden. Die dort dargestellten drei Segel einer sogenannten Aorten-Herzklappe gewährleisten das Schließen und Öffnen, wobei die Segel an ihren freien Rändern unter Bildung eines Überlappungsbereiches im geschlossenen Zustand aneinanderliegen. Die genannte Konstruktion sowie die in der DE 42 22 610 A1 beschriebene Konstruktion lehnen sich eng an die natürliche Aortenklappe an, bei der die Verbindungslinie der Segel mit der natürlichen Aortenwurzel näherungsweise aus der Durchdringung eines Zylinders mit dem Aortengefäß gebildet wird. An diese Bereiche der Verbindungslinie schließen sich stromabseitig Kommissuren an, in denen sich die Linien bzw. die Segel berühren. Diese Kommissuren verhindern ein Durchschlagen der Segel und dienen in Verbindung mit dem Segel-Überlappungsbereich zur gegenseitigen Segelabstützung. Aus vorstehenden Anmerkungen ist offensichtlich, daß die mit dem Stent verbundenen Segel, gleichgültig, ob es sich um eine Herzklappe mit drei oder mit zwei Segeln handelt, an unterschiedlichen Stellen unterschiedlich stark beansprucht werden, wobei nach der Art und Größenordnung der mechanischen Beanspruchung die jeweilige Dicke lokal angepaßt sein sollte. Neben Bereichen größerer Dicke werden hierbei Dicken unter 50 µm gefordert. Abgesehen von der gewünschten Dickenverteilung ist es ferner wesentlich, eine möglichst glatte und homogene freie Segelkante zu erhalten. Sowohl die optimale Dickenverteilung als auch die glatte Segelkante beeinflussen in außerordentlichem Maße die Dauerfestigkeit der Prothese, bei der die Segel Milliarden von Biegelastwechseln standhalten sollen. Spritz- und Tauchverfahren sind zwar grundsätzlich zur Herstellung einer Folie mit lokal unterschiedlichen Dicken geeignet, jedoch liefern diese Verfahren zum Teil nicht die gewünschte reproduzierbare Maßeinhaltung, teils sind auch bei Dicken unterhalb von 100 µm fertigungstechnische Grenzen gesetzt, die sich nur mit extrem hohem apparativen Aufwand überwinden lassen. In jedem Falle unzureichend ist die mit diesen Verfahren herstellbare Qualität der freien Segelkante.

Es ist Aufgabe der vorliegenden Erfindung, ein Verfahren der eingangs genannten Art anzugeben, mit dem - auch bei extrem kleinen Dicken - lokal unterschiedliche Dickenverteilungen einer Folie sowie eine glatte Abschlußkante des ausgeschnittenen Folienstückes schaffen lassen.

Diese Aufgabe wird durch das im Anspruch 1 beschriebene Verfahren gelöst, das erfindungsgemäß dadurch gekennzeichnet ist, daß die Kunststoff-Folie mittels eines Laserstrahlbündels entlang der vorgegebenen Kontur getrennt und/oder flächenweise bis auf das jeweilige gewünschte Dickenmaß abgetragen wird.

Überraschenderweise zeigt sich, daß das flächenweise Abtragen und Trennen mit der aufgabengemäß geforderten Genauigkeit reproduzierbar durchgeführt werden kann, obwohl die Kunststoffoberfläche im jeweiligen Laserbrennfleck lokal aufgeschmolzen und das geschmolzene Material verdampft wird. Der Unterschied des Trennens und des flächenweisen Abtragens mittels Lasers liegt lediglich in der Eindring- bzw. Bearbeitungstiefe, wobei das Trennen bzw. Ausschneiden der gewünschten Kunststoff-Folienkontur durch Ausbildung einer Nut mit wachsender Tiefe erfolgt. Der Laser und die hiermit verbundene Optik als Bearbeitungswerkzeug treten mit dem Werkstück nicht in Kontakt, so daß insoweit auch kein Verschleiß gegeben ist. Über entsprechende Stellmotoren ist eine exakte Führung des Laserstrahlbündels möglich. Darüber hinaus hat das erfindungsgemäße Verfahren den Vorteil, daß hinsichtlich des Kunststoff-Folien-Dickenmaßes bei der Herstellung durch Spritzen, Tauchen oder Aufsprühen keine Aufwendungen hinsichtlich der gewünschten Dickenverteilung erforderlich sind, da die Folien ohnehin einem Abtragungsverfahren unterzogen werden. Dies schließt ein, daß zunächst mit erheblicher verfahrenstechnischer Vereinfachung dickere Folien hergestellt werden können, die dann später entsprechend profiliert und konturiert werden.

Weiterbildungen der Erfindungen sind in den Unteransprüchen beschrieben.

So wird vorzugsweise die Kunststoff-Folie rasterförmig abgetastet, wobei die vorhandenen Dicken punktweise gemessen und gespeichert werden und wonach die gespeicherten Werte zur Ermittlung der zum Trennen und/oder flächenweisen Abtragen notwendigen Laser Einstellparameter wie der Energiedichte, der Pulsrate und der Einwirkdauer verwendet werden. Dieser Maßnahme liegt der Gedanke zugrunde, daß zunächst eine durch beliebige Formgebungsverfahren hergestellte Kunststoff-Folie hinsichtlich der Dickenverteilung vermessen wird, wodurch sich im Vergleich zu der Soll-Dickenverteilung das von Ort zu Ort variierende Maß ergibt, um das die Oberflächenschichten abgetragen werden müssen. In entsprechender Weise ist über die Pulsrate, Laserbrennfleck-Verweildauer sowie die Energiedichte die zulässige Eindringtiefe zu ermitteln, wobei geringere Eindringtiefen, die unterhalb des abzutragenden Maßes liegen, eine längere Verweilzeit des Laserstrahlbündels auf der Werkstückoberfläche oder ein mehrmaliges Überfahren dieses Stückes erfordern. Die flächenhafte Ausdehnung des auf die Werkstückoberfläche einwirkenden Laserstrahlbündels richtet sich im wesentlichen danach, mit welchem Gradienten sich die Dickenverteilung entlang eines Profiles ändert. Eine starke Dickenänderung auf kurzen Strecken erfordert einen in der Flächenausdehnung kleineren Laser-Brennfleck als eine sich nur schwach ändernde Dickenabtragung.

Vorzugsweise wird zum Trennen und/oder flächenweisen Abtragen ein Excimer-Laser, vorzugsweise ein ArF-, KrCl- oder KrF-Laser verwendet. Diese, im UV-Bereich arbeitenden Laser sind Impulslaser, bei denen die Pulsrate und die Pulszahl, die Energiedichte und die Geschwindigkeit, der Grad der Abtragung an der Kunststoffoberfläche einstellbar ist.

Um Reaktionen der während der Laser-Behandlung aufschmelzenden Oberfläche mit der Außenumgebung oder eine Ablagerung von Festkörperpartikeln (Staub) zu verhindern, wird die Kunststoff-Folien-Oberflächenbehandlung vorzugsweise unter einem Druck von 10 Pa bis 10 ⁻¹ Pa, vorzugsweise bei 1 Pa, durchgeführt. Das dementsprechende Vakuum dient gleichzeitig zur Absaugung des oberhalb der Kunststoffoberfläche durch Aufschmelzen entstehenden Dampfdruckes.

Nach einer weiteren Ausgestaltung der Erfindung kann die Oberflächenbearbeitung unter einer O₂- oder einer Schutzgasatmosphäre, die vorzugsweise aus Stickstoff und/oder Helium besteht, durchgeführt werden. Bevorzugt liegt die Laserstrahlbündel-Energiedichte zwischen 0,3 und 1,2 J/cm². Als optimale Pulsfrequenzen haben sich solche zwischen 20 bis 400 Hz, vorzugsweise 20 Hz bis 150 Hz, bewährt. Soweit nicht große Dickengradienten entlang eines Kunststoff-Folien-Profiles zu schaffen sind, wie beispielsweise bei starken Krümmungsradien der Freiformfläche von Segeln in künstlichen Herzklappen, haben sich als Maß für die bestrahlte Kreisfläche Durchmesser zwischen 80 bis 600 µm bzw. rechteckige bestrahlte Flächenstücke mit Kantenlängen von maximal 800 µm bewährt.

Nach einer weiteren Ausgestaltung der Erfindung wird das Laserstrahlbündel zum flächenweisen Abtrag mäanderförmig über die zu bearbeitende Fläche geführt, wobei sich die durch die Führung des Laserstrahlbündels ergebenden Bahnen teilweise geringfügig überlappen. Durch diese Maßnahme wird berücksichtigt, daß die Energiedichte des Laserstrahlbündels zum Rand hin geringer wird, mit der Folge, daß in diesen Randbereichen die Abtragung der Werkstück-Oberfläche geringer ist. Durch die bahnenweise Überlappung ist gewährleistet, daß solche Randbereiche beim nochmaligen "Überfahren" mit dem Laserstrahlbündel auf das gewünschte Dickenmaß abgetragen werden.

Bevorzugt werden die ein- oder mehrlagigen Kunststoff-Folien, die vorzugsweise aus Polyurethan bestehen, durch Spritzgießen, Tiefziehen, Tauchen und/oder Sprühen auf eine Form hergestellt. Das Abtrennen der gewünschten Folienstücke kann durchgeführt werden, während die Folie auf der Form aufliegt. Das Formwerkzeug wird hierbei nicht beschädigt und kann deshalb mehrfach verwendet werden.

Nach einer weiteren Ausgestaltung der Erfindung wird die Kunststoff-Folie vor der Bearbeitung mittels eines Laserstrahlbündels mit einer Schutzschicht überzogen, die nach der Laserbearbeitung wieder entfernt wird. Das mittels des Laserstrahlbündels aus der Schnittfuge bzw. von der Oberfläche der Kunststoff-Folie ausgetragene Material verdampft. Der betreffende Materialdampf ist aus verschiedenen Komponenten zusammengesetzt, die von Bruchstücken der Makromoleküle bis hin zu Partikeln in einer Mikrometer-Größe reichen. Um zu verhindern, daß sich solche Partikel oder andere aus der Dampfphase resultierende Niederschläge als Anhäufung von Partikeln (Debris) unmittelbar auf der Kunststoff-Folie ablagern, wird diese zwischenzeitlich mit einer Schutzschicht, z.B. durch Tauch- oder Sprühverfahren, überzogen. Diese Schutzschicht kann zusammen mit der Debris nach dem Schneiden wieder entfernt werden, so daß die gefertigte Folienkontur und -dicke nach Entfernung der Schutzschicht wieder erhalten wird. Vorzugsweise besteht die Schutzschicht aus wasserlöslichem Material, das nach der Laserbehandlung leicht abgewaschen werden kann.

Nach einer Weiterbildung der Erfindung soll das Schutzschicht-Material bioverträglich sein, d.h. physiologisch unbedenklich, so daß etwa auch nach dem Abwaschen der Schutzfolie verbleibende Reste vom menschlichen Körper ohne Schädigung vollständig abgebaut werden können. Als Schutzschicht-Material bieten sich insbesondere wasserlösliche Polymere mit Molgewichten zwischen 2000 und 150000 mit filmbildenden Eigenschaften an. Bevorzugt verwendete Polymere sind Polyethylenoxid, Polyvinylalkohol, Polyvinylpyrrolidon, Polysaccharide, Polylactide, Polynatriumstyrolsylfonat und/oder Polyacrylsäure sowie Mischungen dieser Substanzen. In Betracht kommen selbstverständlich auch andere wasserlösliche, bioverträgliche und physiologisch unbedenkliche weitere organische Verbindungen.

In einem konkreten Ausführungsbeispiel ist mit dem erfindungsgemäßen Verfahren das Segel einer Aortenherzklappe hergestellt worden. Das erfindungsgemäße Trennverfahren erlaubt das Auftrennen der freien Segelkante bei nahezu beliebiger Raumkurve, wohingegen das flächenweise Abtragen zum Einstellen einer lokal vorgebbaren Segeldicke dient. So ist im Bereich des sogenannten Nodulus Arantii, wo sich die drei Segel beim Klappenschluß treffen, eine vergleichsweise dünne Dicke eingestellt worden, um einerseits einen sicheren Schluß der Klappe zu gewährleisten und andererseits die Biegebeanspruchung gering zu halten.

Zur Herstellung des Polyurethan-Segels ist eine Form verwendet worden, die mehrfach getaucht worden ist, bis die auf der Tauchform gehärtete Schicht eine Dicke aufweist, die an jedem Ort mindestens so groß ist wie die lokal gewünschte Dicke. Sowohl zum Trennen, d.h. zum Ausschneiden der gewünschten Segelkontur, als auch zum flächigen Abtragen der Oberflächenbereiche zur gezielten Dickeneinstellung ist ein ArF-Laser mit einer Wellenlänge von 193 nm verwendet worden. Das Entladungsvolumen ist durch Modenblenden eingeschränkt, so daß Isotropstrahlung mit einer niedrigen Divergenz emittiert wird. Hierdurch wird zwar die Pulsenergie (beim Trennen) auf etwa 10 mJ beschränkt, andererseits die Strahlqualität soweit erhöht, daß unter Verwendung einer einfachen Plankonvexlinse einer Brennweite von 50 mm feine Trennschnitte erzeugt werden können. Die betreffende Kunststoff-Folie wird in eine Vakuumkammer gebracht, die mit einem Suprasilfenster abgedeckt ist. Der Laserstrahl bzw. das Laserstrahlbündel wird in ein Bearbeitungsmikroskop eingekoppelt und mit Hilfe der genannten Linse durch das Fenster als kreisförmiger Fleck mit einem Durchmesser zwischen 90 µm und 600 µm auf die Kunststoff-Folie abgebildet. Die Kammer wird mittels eines gesteuerten Schrittmotors mit konstanter Geschwindigkeit relativ zum Laserstrahl bewegt. In der Kammer, in der die Kunststoff-Folie bearbeitet wird, wird ein Unterdruck von 10² Pa eingestellt und durch weiteres Pumpen aufrechterhalten. Alternativ wird Stickstoff, Sauerstoff und/oder Helium unter einem Druck zwischen 1 bis 10⁴ Pa eingefüllt. Zum Teil werden die Trennarbeiten auch unter Normaldruck bei Luft durchgeführt. Der Abstand zwischen der Kunststoff-Folie und dem genannten Suprasilfenster sollte nicht zu gering gewählt werden, da beim einem Abstand von etwa 20 mm und Drücken <10³ Pa ein gelblich-brauner Niederschlag auf dem Fenster zu beobachten war. Dieser aus der Verdampfung resultierende Niederschlag führt zu schwankenden Transmissionen zwischen 30 und 65 %, auch wenn sich der Laserstrahl "seine Durchtrittsstelle freibrennt". Die Schnittgeschwindigkeit hängt im wesentlichen von der Abtragsrate, d.h. von der erreichbaren Abtragstiefe pro Puls ab, die zwischen 0,05 µm/Puls bis 1 µm/Puls je nach verwendeter Energiedichte zwischen 0,55 J/cm² und 2 J/cm². Die Pulsraten werden zwischen 20 Hz, 100 Hz und 150 Hz variiert; mit Hilfe einer runden Blende werden Strahldurchmesser auf Werte von 90 µm, 225 µm und 600 µm eingestellt.

Das aus der durch das Laserstrahlbündel aus der Schnittfuge der Kunststoff-Folie ausgetragene Material strömt mit einer Vorzugsrichtung senkrecht zur Oberfläche ab. Der betreffende Materialdampf ist aus verschiedenen Komponenten zusammengesetzt, die von Bruchstücken der Makromoleküle bis hin zu Partikeln in einer µm-Größe reichen. Ein Teil dieser Partikel lagert sich neben der Schnittfuge ab und erscheint als bräunlicher Niederschlag, was unter dem Mikroskop als eine Anhäufung von Partikeln (sogenannte Debris) zu erkennen ist. Die typische Partikelgröße beträgt etwa 200 nm. Es stellt sich heraus, daß die Umgebungsbedingungen die Menge und die Art der Ablagerungen beeinflussen. Die besten Ergebnisse können im Vakuum (<10⁴ Pa) erreicht werden, wo das Material ungehindert abströmen kann. Weiterhin günstig wirkt sich die Verwendung von Sauerstoff oder Helium als Spülgas aus.

Wie bereits vorstehend erläutert, wird in einem weiteren Ausführungsbeispiel die verwendete Kunststoff-Folie vor der Behandlung mittels eines Laserstrahlbündels mit einer wasserlöslichen und bioverträglichen Schutzschicht überzogen. Als Schutzschichten sind Polyethylenoxid, Polyvinylalkohol, Polyvinylpyrrolidon, Polysaccharide, Polylactide, Polynatriumstyrolsylfonat oder Polyacrylsäure sowie Mischungen dieser Substanzen verwendet worden. Die Dicke dieser Schicht braucht nur wenige Mikrometer betragen (vorzugsweise bis maximal 10 µm), da diese Schutzschicht lediglich dazu dient, die Partikel-Niederschläge "abzufangen", d.h. um zu verhindern, daß sich diese Partikel-Niederschläge auf der Folie selbst ablagern. Nach dem Schneiden oder Oberflächenbearbeiten der Kunststoffolie wird die Schutzfolie, die wasserlöslich ist, wieder abgewaschen, wobei gleichzeitig die Partikel entfernt werden. Auf diese Weise kann das gefertigte Dickenmaß bzw. die Kunststoff-Folienoberfläche erhalten bleiben.

Die Schnitte können in einem einzigen Arbeitsgang oder durch zwei- oder mehrfaches Überfahren einer Spur, welche gleichzeitig die Trennlinie ist, erreicht werden.

Zum flächigen Abtragen der Kunststoff-Oberfläche wird ein Laserstrahlbündel verwendet, dessen Flächenmaß auf der Kunststoff-Oberfläche ca. 400 x 800 µm beträgt. Das Laserstrahlbündel wird parallel zu seiner Kante über den Kunststoff-Oberflächenfilm bewegt, wobei der Laser mit einer festen Pulsfrequenz betrieben wird. Nach einer vorgegebenen Strecke wird der Laserstrahl zur Seite versetzt und parallel wieder zurückbewegt, wobei sich die überstrichenen Flächenbereiche am Rand überlappen. An den Umkehrpunkten wird der Laser mit seiner Pulsfrequenz entsprechend der Versatzgeschwindigkeit synchronisiert. Durch das mäanderförmige Durchlaufen mehrerer parallel zueinander verlaufender Spuren wird die gesamte Kunststoff-Oberfläche abgeschritten. Die Parameter, Energiedichte, Pulsfrequenz und Pulszahl werden nach Vorgabe der zuvor eingescannten Dickenmaße derart variiert, daß an jeder Stelle der Polyurethan-Folie nach dem Abtrag die gewünschte Dicke erreicht wird. Unabhängig von der Pulszahl wird mit 20 Hz-Pulsraten bei Energiedichten von 0,17 bis 0,93 J/cm² gearbeitet. In einer weiteren Reihe sind die Pulsraten bis 80 Hz bei Energiedichten zwischen 0,37 bis 1,1 J/cm² variiert worden.

Die Versuche haben ergeben, daß das Auftreten von Blasen, Kegeln oder Rillen bei Energiedichten von mehr als 0,2 J/cm², vorzugsweise von mehr als 0,4 J/cm², verwendet werden. Die Pulsrate liegt vorzugsweise unter 80 Hz.

Mit dem genannten Verfahren läßt sich lokal eine geringe Dicke von weniger als 50 µm gezielt einstellen. Obgleich sowohl das Trennen der PU-Folie als auch das flächige Abtragen mit einem runden oder einem rechteckigen Laserstrahlbündel durchführbar ist, wird ein rundes Bündel bevorzugt.

Die Dickenverteilung eines Segels wird vorzugsweise so gewählt, daß sie der Dickenverteilung eines natürlichen Segels entspricht. Eine solche Dickenverteilung für Aorten-Segel wird z.B. in Richard E. Clark et al. "Leaflet Prosthetic Valves, Cardiovascular Diseases", Vol. 1, No. 3, 1974, Seite 437, angegeben und liegt zwischen 0,25 mm und 1,1 mm. Wegen der härteren verwendeten Kunststoff-Folien, z.B. aus PU, entspricht dies einer Kunststoff-Segel-Dickenverteilung zwischen 50 und 250 µm.

## Patentansprüche

1. Verfahren zur Herstellung von Kunststoff-Folien mit einer vorgebbaren Kontur und/oder einer gleichmäßigen oder ungleichmäßigen Dickenverteilung durch trennende und/oder abtragende Bearbeitung von vorgefertigten Kunststoff-Folien, insbesondere zur Herstellung von flexiblen Segeln für künstliche Herzklappen,
**dadurch gekennzeichnet,**
**daß** die Kunststoff-Folie mittels eines Laserstrahlbündels entlang der vorgegebenen Kontur getrennt und/oder flächenweise bis auf das jeweilige gewünschte Dickenmaß abgetragen wird.

2. Verfahren nach Anspruch 1, **dadurch gekennzeichnet, daß** die Kunststoff-Folie rasterförmig abgetastet wird, wobei die vorhandenen Dicken punktweise gemessen und gespeichert werden und wonach die gespeicherten Werte zur Ermittlung der zum Trennen und/oder flächenweisen Abtragen notwendigen Lasereinstellparameter, wie der Energiedichte, der Pulsrate und der Einwirkdauer verwendet werden.

3. Verfahren nach Anspruch 1 oder 2, **dadurch gekennzeichnet, daß** zum Trennen und/oder Abtragen ein Excimer-Laser, vorzugsweise ein ArF-, KrCL- oder KrF-Laser verwendet wird, wobei vorzugsweise die Laserstrahlbündelenergiedichte zwischen 0,3 und 1,2 J/cm² und die verwendeten Pulsfrequenzen zwischen 20 bis 150 Hz liegen.

4. Verfahren nach Anspruch 3, **dadurch gekennzeichnet, daß** das Trennen und/oder Abtragen unter einem Druck von 10 Pa bis 10⁻¹ Pa, vorzugsweise 1 Pa, durchgeführt wird.

5. Verfahren nach einem der Ansprüche 1 bis 4, **dadurch gekennzeichnet, daß** das Trennen und/oder Abtragen unter einer O₂- oder einer Schutzgasatmosphäre, vorzugsweise aus Stickstoff und/oder Helium durchgeführt wird.

6. Verfahren nach einem der Ansprüche 3 bis 5, **dadurch gekennzeichnet, daß** die zum Trennen und/oder Abtragen momentan bestrahlte Fläche kreisförmig mit einem Durchmesser von 80 bis 600 µm oder rechteckig mit Kantenlängen von maximal 800 µm ist.

7. Verfahren nach einem der Ansprüche 1 bis 6, **dadurch gekennzeichnet, daß** das Laserstrahlbündel zum flächenweisen Abtrag mäanderförmig über die zu bearbeitende Fläche geführt wird, wobei sich die durch die Führung des Laserstrahlbündels ergebenden Bahnen teilweise geringfügig überlappen.

8. Verfahren nach einem der Ansprüche 1 bis 7, **dadurch gekennzeichnet, daß** die ein- oder mehrlagige Kunststoff-Folie, vorzugsweise aus Polyurethan, durch Spritzgießen, Tiefziehen, Tauchen und/oder Sprühen auf eine Form hergestellt und anschließend flächenweise abgetragen und/oder geschnitten wird, wobei vorzugsweise die Kunststoff-Folie vor der Bearbeitung mittels eines Laserstrahlbündels mit einer Schutzschicht überzogen wird, die nach der Bearbeitung mittels des Laserstrahlbündels wieder entfernt wird.

9. Verfahren nach Anspruch 8, **dadurch gekennzeichnet, daß** die Schutzschicht aus wasserlöslichem Material besteht, das nach der Laserbearbeitung abgewaschen wird, und/oder bioverträglich ist, wobei die Schutzschicht vorzugsweise aus wasserlöslichen Polymeren mit Molgewichten zwischen 2000 und 150000 besteht.

10. Verfahren nach Anspruch 9, **dadurch gekennzeichnet, daß** die Schutzschicht aus Polyethylenoxid, Polyvinylalkohol, Polyvinylpyrrolidon, Polysaccharide, Polylactide, Polynatriumstyrolsylfonat und/oder Polyacrylsäure oder Mischungen hieraus besteht.

## Claims

1. Method for producing plastic films with a predetermined contour and/or an uniform or nonuniform thickness distribution, by separating and/or ablating machining of prefabricated plastic film, especially in the production of flexible leaflets for artificial heart valves,
**characterised in that**
the plastic film is separated along the predetermined contour by means of a laser beam bundle and/or is area-wise ablated to the respective desired thickness measurement by means of a laser beam bundle.

2. Method according to claim 1 **characterised in that** the plastic foil is sensed in a scanning manner whereby the existing thickness is machined area-wise and stored, whereupon the stored values are used to determine the laser adjustment parameters being for example, the energy density, the pulse rate and the effective duration.

3. Method according to claim 1 or 2, **characterised in that** for the separation and/or ablation an eximer laser, preferably ArF, KrCl or KrF laser is used, whereby preferably the laser beam bundle energy density lies between 0,3 and 1,2 J/cm² and the pulse frequency is between 20 to 150 Hz.

4. Method according to claim 3, **characterised in that** the separation and/or ablation is carried out under a pressure of 10 Pa to 10⁻¹ Pa, preferably 1 Pa.

5. Method according to one of claims 1 to 4, **characterised in that** the separation and/or ablation is carried out under an O₂ or a protected gas atmosphere, preferably of nitrogen and/or helium.

6. Method according to one of claims 3 to 5, **characterised in that** for the separation and/or ablation an instantaneous surface is irradiated which is circular with a diameter of 80 to 600 µm or rectangular with side length of at most 800 µm.

7. Method according to one of claims 1 to 6, **characterised in that** the laser beam bundle for area-wise ablation is moved in a meander pattern over the surface to be machined, whereby the path of the laser beam bundle resulting from this displacement party and slightly overlap.

8. Method according to one of claims 1 to 7, **characterised in that** the single or multilayer plastic film is made, preferably from polyurethane, by injection moulding, deep drawing, immersion, and/or spraying on a form and then is ablated area-wise and/or cut out, whereby preferably before machining by means of a laser beam bundle the plastic foil is coated with a protective layer which is removed after machining by means of the laser beam bundle.

9. Method according to claim 8, **characterised in that** the protective layer is comprised of water-soluble material which is washed away after the laser machining and/or is biocompatible, whereby the protective layer is preferably comprised of water-soluble polymers with a molecular weight between 2000 and 150000.

10. Method according to claim 9, **characterised in that** the protective layer is composed of polyethyleneoxides, polyvinylalcohols, polyvinylpyrrolidones, polysaccharides, polylactides, polysodimstyrenesulfates and/or polyacrylic acids or mixtures thereof.

## Revendications

1. Procédé de fabrication de feuilles en matière synthétique avec un contour qui peut être prédéterminé et/ou avec une répartition uniforme ou non-uniforme de l'épaisseur, par un usinage de séparation et/ou d'ablation de feuilles en matière synthétique préfabriquées, en particulier pour la fabrication de voiles flexibles pour des valvules artificielles du coeur,
**caractérisé par le fait**
**que** la feuille en matière synthétique est séparée au moyen d'un faisceau de rayons laser le long du contour prédéterminé et/ou est ablatée par surfaces au moyen d'un faisceau de rayons laser jusqu'à ce qu'elle présente la mesure d'épaisseur souhaitée respective.

2. Procédé selon la revendication 1, **caractérisé par le fait que** la feuille en matière synthétique est balayée en forme de trame, les épaisseurs existantes étant mesurées par points et mémorisées, et, après quoi, les valeurs mémorisées sont utilisées pour déterminer les paramètres de réglage du laser tels que la densité de l'énergie, le taux d'impulsions et la durée d'action, qui sont nécessaires à la séparation et/ou à l'ablation par surfaces.

3. Procédé selon la revendication 1 ou 2, **caractérisé par le fait que**, pour la séparation et/ou l'ablation, on utilise un laser excimer, de préférence un laser ArF, KrCL ou KrF, de préférence la densité de l'énergie du faisceau de rayons laser étant comprise entre 0,3 et 1,2 J/cm² et les fréquences utilisées de répétition des impulsions étant comprises entre 20 et 150 Hz.

4. Procédé selon la revendication 3, **caractérisé par le fait que** la séparation et/ou l'ablation est ou bien sont réalisée(s) sous une pression de 10 Pa à 10⁻¹ Pa, de préférence de ¹Pa.

5. Procédé selon l'une des revendications 1 à 4, **caractérisé par le fait que** la séparation et/ou l'ablation est ou bien sont réalisée(s) sous une atmosphère de O₂ ou de gaz protecteur, de préférence en azote et/ou en hélium.

6. Procédé selon l'une des revendications 3 à 5, **caractérisé par le fait que** la surface qui, pour la séparation et/ou l'ablation, est soumise momentanément au rayonnement est circulaire avec un diamètre de 80 à 600 µm ou rectangulaire avec des longueurs d'arête de 800 µm au maximum.

7. Procédé selon l'une des revendications 1 à 6, **caractérisé par le fait que**, pour l'ablation par surfaces, le faisceau de rayons laser est guidé en forme de méandre sur la surface à usiner, les trajectoires résultant du guidage du faisceau de rayons laser se recouvrant en partie légèrement.

8. Procédé selon l'une des revendications 1 à 7, **caractérisé par le fait que** la feuille à une ou plusieurs couches en matière synthétique, de préférence en polyuréthane, est fabriquée par moulage par injection, par emboutissage, par plongée et/ou par pulvérisation sur une forme et est ensuite ablatée par surfaces et/ou est coupée, de préférence, avant l'usinage au moyen d'un faisceau de rayons laser, la feuille en matière synthétique étant recouverte dune couche protectrice qui, après l'usinage au moyen du faisceau de rayons laser, est de nouveau enlevée.

9. Procédé selon la revendication 8, **caractérisé par le fait que** ladite couche protectrice se compose d'une matière soluble dans l'eau qui est enlevée par lavage après l'usinage au moyen du laser et/ou est biocompatible, la couche protectrice se composant de préférence de polymères solubles dans l'eau, avec des poids molaires compris entre 2000 et 150000.

10. Procédé selon la revendication 9, **caractérisé par le fait que** ladite couche protectrice se compose de polyéthylène oxyde, d'alcool polyvinylique, de polyvinylpyrrolidone, de polysaccharides, de polylactides, de polysodiumstyrènesylfonate et/ou d'acide polyacrylique ou de mélanges de ceux-ci.
